(19) Europäisches Patentamt

European Patent Office

Office européen des brevets

(11) **EP 0 562 034 B1**

(12) **EUROPEAN PATENT SPECIFICATION**

(45) Date of publication and mention
of the grant of the patent:
**16.10.1996 Bulletin 1996/42**

(21) Application number: **92903831.3**

(22) Date of filing: **27.11.1991**

(51) Int Cl.[6]: **C07C 5/44**, C07C 17/10,
C07C 11/04, C07C 21/06,
C07C 17/156, C07C 19/045,
C07C 1/30

(86) International application number:
**PCT/US91/08754**

(87) International publication number:
**WO 92/12946 (06.08.1992 Gazette 1992/21)**

(54) **PRODUCTION OF ALKENES**

HERSTELLUNG VON ALKENEN

PRODUCTION D'ALCENES

(84) Designated Contracting States:
**AT BE CH DE DK ES FR GB GR IT LI LU NL SE**

(30) Priority: **06.12.1990 US 621719**

(43) Date of publication of application:
**29.09.1993 Bulletin 1993/39**

(73) Proprietor: **UNIVERSITY OF SOUTHERN CALIFORNIA**
**Los Angeles, CA 90007-4344 (US)**

(72) Inventors:
• **BENSON, Sidney, W.**
**Los Angeles, CA 90049 (US)**

• **WEISSMAN, Maja, A.**
**Santa Clarita, CA 91350 (US)**

(74) Representative: **UEXKÜLL & STOLBERG**
**Patentanwälte**
**Beselerstrasse 4**
**22607 Hamburg (DE)**

(56) References cited:
**DE-B- 1 114 184**      **FR-A- 2 248 254**
**GB-A- 508 417**        **US-A- 3 862 996**

Remarks:
The file contains technical information submitted after the application was filed and not included in this specification

## Description

This invention relates to a process for making ethylene and mixtures of ethylene and vinyl chloride. More particulary, the invention relates to a novel process for making ethylene and mixtures of ethylene and vinyl chloride by the reaction of ethane with chlorine.

Ethylene is a valuable and widely used commodity. Over ten billion pounds of ethylene are consumed each year in the United States alone, to make various grades of polyethylene.

Another major use of ethylene is as the starting material for making vinyl chloride, which can then be polymerized into polyvinyl chloride (PVC).

In view of the huge quantities of ethylene consumed each year, there is substantial interest in any economical and improved method for making ethylene. At the present time, ethylene is typically made by the high temperature dehydrogenation of ethane and cracking feedstocks such as propane, butane, and naphtha. Such high temperature processes require the expenditure of substantial amounts of energy, which is expensive.

There have been a number of attempts to develop a viable process for the dehydrogenation of ethane or other lower olefinic hydrocarbons by reaction with chlorine or chlorine-containing compound at lower temperatures. As far as we know, none of these prior attempts have resulted in a commercially viable process.

Baehr (U.S. Patent No. 2,259,195) discloses a process in which chlorine is used to dehydrogenate paraffinic and olefinic hydrocarbons having 3 to 8 carbon atoms. In this process the chlorine and the hydrocarbon are mixed and reacted in the gas phase at a temperature of 300°C to 800°C. In Comparative Example A herein, we show that the procedure of Baehr's Example 1, when applied to ethane, would cause almost immediate coking and plugging of the system.

Gorin et al U.S. 2,488,083 shows a process for converting gaseous methane and natural gas to liquid hydrocarbons via alkyl halide intermediary, followed by dehydrohalogenocondensation. The separation of hydrogen chloride from other gaseous materials is shown.

Dirstine et al (U.S. Patent No. 2,628,259) discloses a process for chlorinating ethane to produce vinylidene chloride (1,1-dichloroethylene) and vinyl chloride. This process is conducted at a temperature of 450°C to 600°C, in the presence of a diluent gas at a chlorine to ethane molar ratio of between 1.9:1 and 3.0:1.

Conrad (U.S. Patent No. 2,838,579) discloses a process for the chlorination of ethane to produce chloroethane products such as ethyl chloride, 1,1-dichloroethane or 1,2-dichloroethane, or higher chloroethanes if desired. The process is conducted at high pressure in a temperature range of 300 to 600°C in the presence of a fluidized bed catalyst consisting of inorganic, carbon coated particles.

Mullineaux (U.S. Patent No. 2,890,253) discloses the use of iodine and oxygen to dehydrogenate saturated hydrocarbons including ethane to yield unsaturated hydrocarbons. In the example showing reaction of iodine with ethane (Example VI, column 10) the amount of ethane reacted was only 40 percent.

Taylor (U.S. Patent No. 3,166,601) discloses a process for the chlorination of ethane to produce unsaturated, chlorinated products. This process is conducted at a temperature of 600 to 900°C in the presence of an inert diluent gas.

Carroll (U.S. Patent No. 3,173,962) discloses a process for converting alkanes containing 2 to 6 carbon atoms into olefins and chlorinated alkanes which comprises passing a mixture of an alkane, hydrogen chloride, and oxygen or oxygen-containing gas over a catalyst, at a temperature of about 300 to 650°C.

Bajars discloses (U.S. Patent No. 3,207,811) a catalytic process for dehydrogenating aliphatic hydrocarbons of 4 to 5 carbon atoms which comprises heating an aliphatic hydrocarbon of 4 to 6 carbon atoms with oxygen, and a source of chlorine to a temperature of at least 450°C up to 1000°C in the presence of a catalyst.

Riegel (U.S. Patent No. 3,557,229) discloses a catalytic process for the oxychlorination of ethane to produce vinyl chloride, along with ethyl chloride, dichloroethane, ethylene and other compounds. The reactants are ethane, hydrochloric acid and an oxygen source.

FR-A-2 248 254 discloses a process for preparing ethylene and vinyl chloride from ethane and chlorine wherein the reactants are preheated to temperatures in the range of 400 to 500 °C prior to their entering into the reaction zone.

Beard (U.S. Patent No. 3,558,735) discloses a catalytic oxydehydrogenation process for the production of ethylene in which ethane is reacted with hydrogen chloride and oxygen in the presence of a fluidized copper chloride and rare earth halide catalyst at a temperature of above 350°C to about 650°C.

Beard discloses (U.S. Patent Nos. 3,658,934; 3,702,311; and 3,862,996) a catalytic process for the production of ethylene which comprises reacting ethane with a halogen, in the presence of an inert gas diluent and a catalyst at a temperature of above 350°C to about 650°C.

Kroenke et al disclose in a series of patents (U.S. Patent Nos. 4,119,570; 4,375,569; 4,461,919; and 4,467,127) a process for the oxychlorination of ethane to produce a mixture of ethylene, ethylene dichloride, vinyl chloride, and ethyl chloride. In this process ethane, oxygen, preferably from air, and a chlorine source such as hydrogen chloride are reacted in the presence of a catalyst at a temperature from 400°C to about 650°C.

Zaidman et al (U.S. Patent No. 4,217,311) disclose a process for the production of vinyl chloride. In this process, a mixture of ethylene and ethane are reacted with chlorine at a temperature of beeveen 300°C and 550°C. The chlorine is added at several points along the reaction zone.

Li (U.S. Patent No. 4,300,005) discloses a catalytic process for producing monohalogenated olefins and other products by the oxychlorination of 2 to 4 carbon alkanes. In the process the alkane is reacted with a hydrogen halide, and an oxygen source at a temperature of about 400°C to 650°C in the presence of a catalyst.

Pyke et al (British Patent Nos. 2,095,242A and 2,095,245A) disclose a catalytic process for producing vinyl chloride by reacting ethane with a chlorine source and oxygen at a temperature of 275°C to 500°C.

## Summary of the Invention

In accordance with the present invention, there is provided a process for reacting ethane with chlorine to produce a mixture of ethylene and vinyl chloride. This process is characterized by a conversion of ethane per pass through the reactor of about 50% or better and a combined molar yield of ethylene and vinyl chloride of over about 80% of the ethane consumed. In conducting the process of this invention, ethane is intimately mixed with chlorine and the mixture is heated in an inert reactor to initiate the reaction. The reaction is allowed to proceed to completion, and the reacted mixture leaving the reaction zone will have a temperature of between about 600°C and about 800°C. The ratio of ethane to chlorine is about 0.9:1 or above.

## The Drawings

Figure 1 is a graph showing the yield of ethylene in the process of this invention as a function of the ratio of ethane to chlorine in the feed and of the reaction temperature;

Figure 2 is a graph showing the yield of vinyl chloride as a function of the ratio of ethane to chlorine in the feed and as a function of the reaction temperature;

Figure 3 is a graph showing the yield of ethyl chloride in the process of this invention as a function of the ratio of ethane to chlorine in the feed and as a function of the reaction temperature; and

Figure 4 is a graph showing ethane conversion, and yields of ethylene, vinyl chloride, and ethyl chloride, as a function of reaction time.

Figure 5 is a schematic diagram of the process of the invention.

## Detailed Description of the Invention

The present invention provides an efficient process for the production of ethylene or a mixture of ethylene and vinyl chloride by reacting chlorine with ethane.

In accordance with this invention, there is provided a process for preparing ethylene or a mixture of ethylene and vinyl chloride by reaction of ethane and chlorine which comprises:

(a) providing a stream of ethane feed gas and a stream of chlorine feed gas;

(b) thoroughly mixing said ethane and chlorine feed gases in a mixing zone at a molar ratio of ethane to chlorine of at least about 0.9;

(c) controlling the temperatures of said ethane feed gas and said chlorine feed gas so that substantially no chemical reaction takes place during and immediately after said mixing and such that the temperature of such mixture of gases immediately after mixing is less than about 215°C;

(d) passing said mixture into an inert reaction zone;

(e) heating said mixture in said inert reaction zone to initiate chemical reaction between said ethane and said chlorine this reaction being uncatalysed;

(f) continuing the providing of heat to said reacting mixture as required so that the reacted mixture will have a temperature between about 600°C and about 800°C;

such that the combined molar yield of ethylene and vinyl chloride is at least about 80% of the ethane consumed.

It is believed that the conversion of ethane and chlorine to produce ethylene and vinyl chloride results from a series of several intermediate reactions, including:

(1) chlorination of ethane to form ethyl chloride as represented by the equation:

$$C_2H_6 + Cl_2 \rightarrow C_2H_5I + HCl$$

(2) cracking of ethyl chloride to form ethylene as represented by the equation:

$$C_2H_5Cl \rightarrow C_2H_4 + HCl$$

(3) chlorination and dehydragenation of ethane to form vinyl chloride through a series of reactions which can be represented by the overall equation:

$$C_2H_6 + 2Cl_2 \rightarrow C_2H_3Cl + 3HCl$$

(4) dehydrogenation of ethane to form ethylene, as represented by the equation:

$$C_2H_6 \rightarrow C_2H_4 + H_2$$

Of these reactions, the chlorination of ethane (reaction (1)) is highly exothermic, generating 28.65 kcal/mole of ethyl chloride formed. The heat of reaction may be relied on, at least in part, in the process of this invention to heat the mixture of ethane and chlorine to the desired reaction temperature. Reaction (3) is also exothermic, generating 41 kcal/mole of vinyl chloride formed, and may also provide heat to sustain the process of this invention. Reactions (2) and (4), on the other hand, are endothermic, and require 17.27 kcal/mole of ethyl chloride reacted, and 32.74 kcal/mole of ethane reacted, respectively.

The process comprises a reaction wherein the reacted mixture has a temperature between 600°C and 800° C, and further comprising cracking the ethyl chloride in said reacted mixture to ethylene and hydrogen chloride.

Said cracking is preferably carried out at about 500°C in the presence of a dehydrohalogenation catalyst.

According to the present invention, ethane is mixed with gaseous chlorine at a temperature such that the two gases do not react. It is believed that reaction (1) is a free radical reaction initiated by thermal dissociation of chlorine into free radicals which then initiate the chlorination of ethane. Since such free radicals are known to be formed at a temperature of at least about 215°C, the ethane and chlorine are mixed at a temperature below 215°C. Although the ethane and chlorine may be conveniently mixed at ambient temperatures, these reactants may also be mixed at somewhat higher temperatures so long as the chlorine does not exceed about 200°C to 215°C either before or immediately after the mixing. Thus, for example, the ethane supplied to the mixing zone may have a temperature of about 200°C and chlorine a temperature of about 20°C, resulting in a mixture at about 180°C, due to the molar ratio of ethane and chlorine employed and their heat capacities. It is important that the chlorine be in the gaseous form and that no liquid chlorine be present, which can lead to undesirable explosions.

The ratio of chlorine to ethane in the feed mixture should be selected carefully in conducting the process of this invention. The higher the ratio of ethane to chlorine, the less vinyl chloride will be poduced. If too little chlorine is used, the reaction will produce few by-products and little vinyl chloride, but will leave a large amount of ethane unreacted. Consequently, more elaborate product separation will be required to recover the ethylene and recycle the unreacted ethane. On the other hand, the presence of too much chlorine will lead to polychlorinated products, other side products, and carbon formation. We have found that good results are obtained with a mole ratio of ethane to chlorine of at least about 0.9:1, and more generally in a range of from about 1:1 to about 4:1. The preferred range of the ratio of ethane to chlorine is from about 1:1 to about 2:1, with a ratio of from about 1.3:1.0 to about 1.6:1.0 being especially preferred.

The mixing of the ethane and chlorine may be performed by any procedure which produces an intimate mixture of the gases. If mixing is incomplete when the gas mixture is conducted to the reactor, undesired reactions will occur in regions where there is a local excess of chlorine, which can lead to the formation of polychlorinated compounds, acetylene, and even carbon. We have found that passing the gases through a packed column produces the desired mixing. However, other methods of mixing will be readily apparent to those skilled in the art.

After mixing the ethane and chlorine, the mixture is conducted to an inert reactor, where heat is added to the mixture. At about 215°C, an exothermic reaction between ethane and chlorine to produce ethyl chloride begins, and causes a further temperature rise. At approximately 600°C, the endothermic dehydrohalogenation to form ethylene occurs with some rapidity. Consequently, in order to achieve the objects of the present invention, the temperature within the reaction zone should be above about 600°C. It is preferred that the highest temperature in the reaction zone not exceed 800°C for a substantial period of time. The temperature may exceed 800°C if the period of high temperature is short, on the order of 1 second or so. The reaction is preferably carried out at a temperature of from about 600°C to about 800°C, and a final temperature of the reacted mixture of from about 650°C to about 750°C is particularly preferred.

In order to achieve the purposes of this invention, it is important that the inner surface of the reactor be inert. Most metallic reactors cause side reactions which lead to carbon formation. We have found that quartz, silicon carbide, alumina, and graphite linings are suitable. However, one skilled in the art could, without undue experimentation, find other inert materials which would be suitable for the lining of a reactor for this process.

The inert reactor of this invention may take many forms, such as a tubular reactor, a cylindrical reactor, or a spherical reactor. It may be a single reactor or it may be divided into several reaction chambers. Each separate reaction chamber may be heated at the same rate or at different rates. The single reactor may contain regions which are heated at different rates. Optionally, the gases may pass through an unheated zone in a single reactor or an unheated chamber in a multiple chamber reactor. In this zone or chamber, the heats of reaction of the exothermic and endothermic reactions control the reaction temperature. If the reactor is a tubular reactor of a diameter small enough to cause plug flow, in the absence of heating or cooling there will be a distinct temperature profile from inlet to outlet. That is, once a reaction is initiated at about 215°C or above, the temperature will rise due to exothermic reaction (1). Then the reaction temperature will peak and decrease as exothermic reaction (1) terminates and endothermic reactions, such as reactions (2) and (4), take place. In such a reactor, heat may be applied in the first stage to initiate the reaction and in the final stage to drive the endothermic reactions and maintain the reaction temperature in the range of 600°C to 800°C. Reaction time is usually less than one minute. It has been found, however, that reaction times of at least 6 to 8 seconds are desired to minimize the formation of ethyl chloride.

The process of this invention is not limited to atmospheric pressure, and it may be carried out at pressures as high as about 30 atmospheres. In selecting a pressure, one must balance the greater throughput at higher pressure against the extra strength required in a high pressure reactor. It is preferred, however, to run close to atmospheric pressure i. e., less than 2 atmospheres.

In practice, the process of this invention does not convert 100% of the ethane entering the reactor. Accordingly, the product stream from the process consists predominantly of ethylene, hydrogen chloride, some hydrogen, and unreacted ethane. The reactant gas stream may be readily fractionated, by methods well-known to those skilled in the art, to separate the various components. This is not necessary, however, and the hydrogen chloride and the ethylene may be processed together to yield 1,2-dichloroethane and vinyl chloride. In these reactions, small amounts of hydrogen and ethane do not present a problem.

Oxychlorination reactions are known, in which ethylene, hydrochloric acid and an oxygen source (generally air or pure oxygen) are reacted to form 1,2-dichloroethane. The overall equation for this reaction is:

$$C_2H_4 + 2HCl + 1/2O_2 \rightarrow C_2H_4Cl_2 + H_2O$$

If the stream of product gases does not contain an appropriate balance of ethylene and hydrogen chloride, one or the other of the reactants may be added, or, alternatively, removed. Such reactions are usually conducted in a temperature range of 225°C to 250°C over a catalyst such as copper chloride on alumina. The product of this reaction is 1,2-dichloroethane which, in turn, may be thermally cracked to yield vinyl chloride.

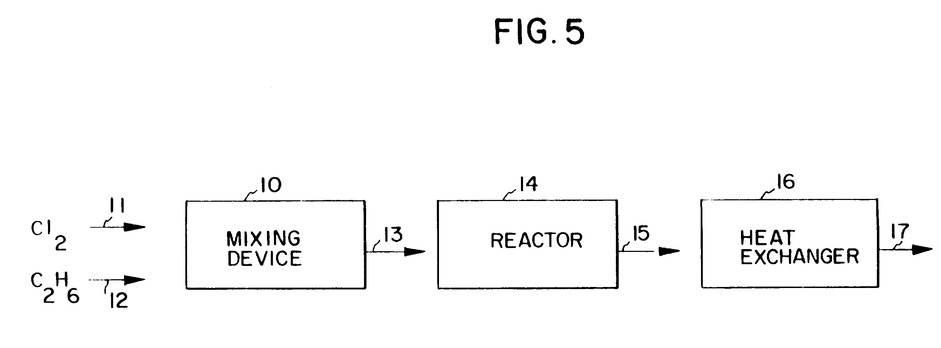

The process of the present invention is further illustrated by the schematic drawing of Figure 5. In Figure 5, a feed stream of chlorine 11 and a feed stream of ethane 12, which may be both at substantially ambient temperature, are fed into a mixing device 10 to produce a thoroughly mixed gaseous stream 13. Feed stream 13 is then fed into a reactor 14 equipped with heating device for raising the temperature of the mixture of chlorine and ethane to at least about 215°C to initiate the reaction. Reactor 14 should provide sufficient heat to the reacting mixture so that the reacted mixture is will leave reactor 14 at about 600°C to about 800°C. The reacted mixture 15 may then be passed through a heat exchanger 16 to quench the mixture to a cooled stream 17 for further separation and processing. As indicated above, the inner surface of reactor 14 should be inert and non-metallic, preferably made of such materials as quartz, silicon carbide, alumina or graphite linings.

The following examples, unless indicated otherwise, illustrate specific embodiments of the invention, but should be construed as merely illustrative, and not limiting of the present invention.

Example 1

A series of experiments was performed in which mixtures of ethane and chlorine at ambient temperature containing ratios of ethane to chlorine varying from about 0.94:1 to about 3.33:1, were fed to a preheated quartz tubular reactor 140cm long and having an inner diameter of 8cm. Reactions of ethane and chlorine were carried out at 625°C, 675°C, and 740°C, as determined from the exterior surface of the middle of the reactor (the so-called "mid-skin temperature"). Residence time in the reactor was about 4 seconds. The reaction product was air quenched and water cooled and, after equilibrium was established, analyzed by gas chromatography The results were as follows.

TABLE I

| Yields of Ethylene, Vinyl Chloride and Ethyl Chloride As A Function of Temperature And Ethane/Chlorine Mole Ratio | | | | |
|---|---|---|---|---|
| Reactions Conditions | | Yield/% | | |
| Temp/°C | Ethane/Chlorine Mole Ratio | Ethylene | Vinyl Chloride | Ethyl Chloride |
| 740 | 1.08:1 | 72.4 | 13.7 | 0 |
| | 1.61:1 | 84.4 | 7.3 | 0 |
| | 2.43:1 | 88.1 | 3.2 | 0 |
| | 3.21:1 | 88.5 | 2.5 | 0 |
| 675 | 1.04:1 | 67.0 | 24.6 | 3.3 |
| | 1.68:1 | 78.9 | 13.3 | 4.8 |
| | 2.48:1 | 82.4 | 9.4 | 4.6 |
| | 3.31:1 | 80.6 | 6.7 | 9.5 |
| 625 | 0.94:1 | 62.1 | 28.5 | 5.5 |
| | 1.65:1 | 53.3 | 15.2 | 30.1 |
| | 2.61:1 | 46.0 | 8.1 | 43.0 |
| | 3.33:1 | 44.4 | 6.7 | 47.2 |

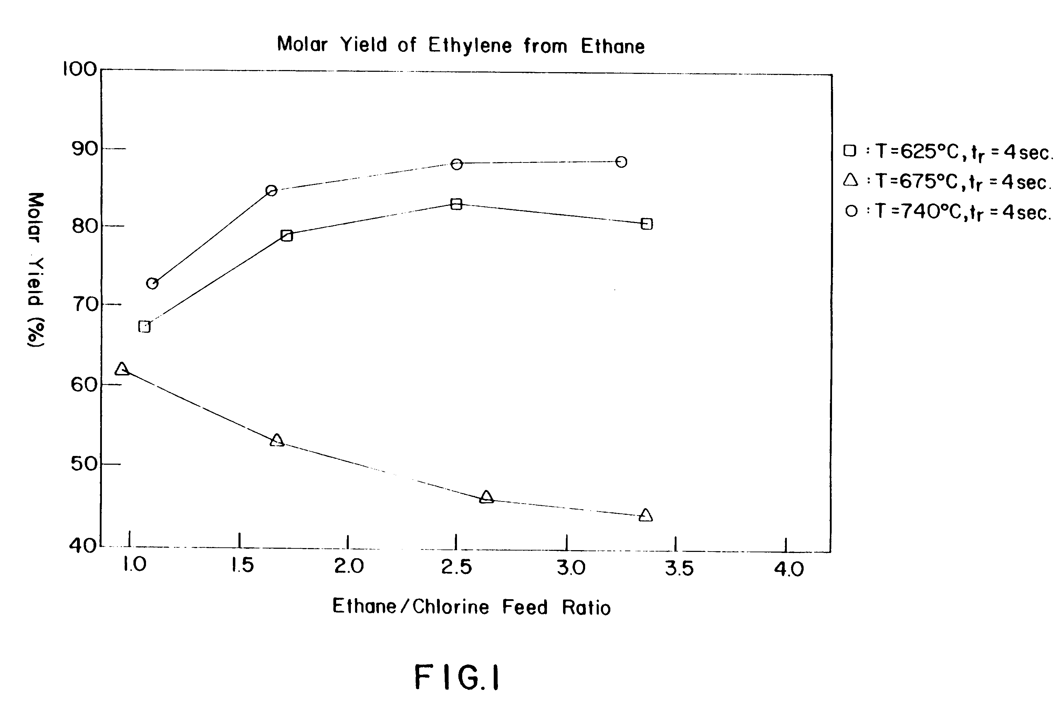

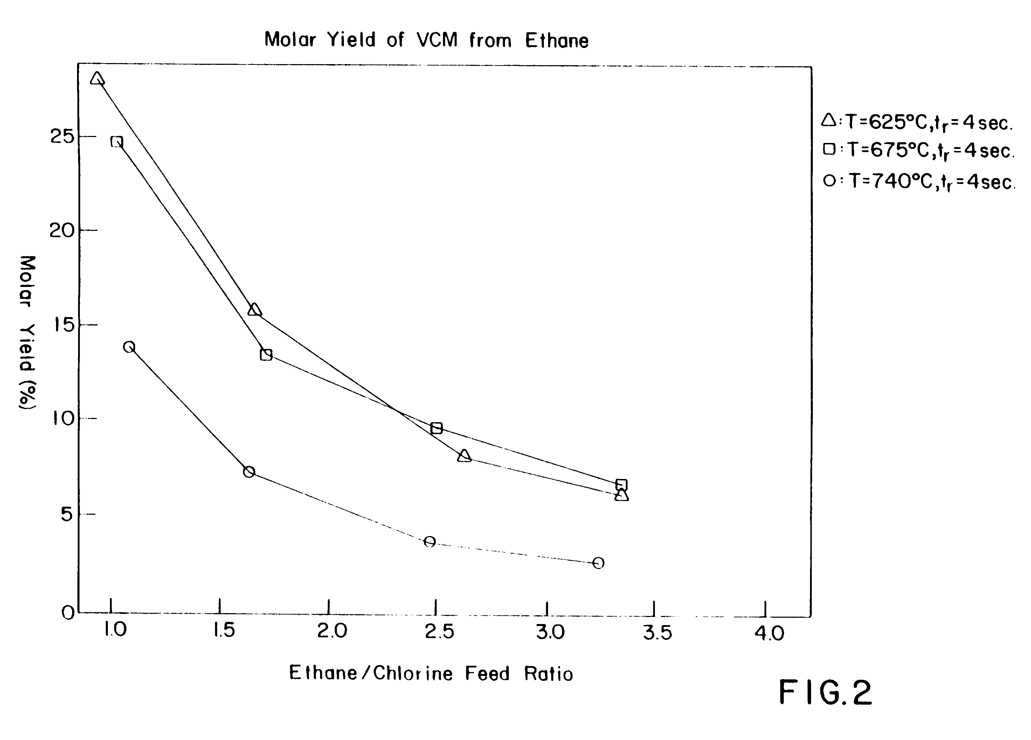

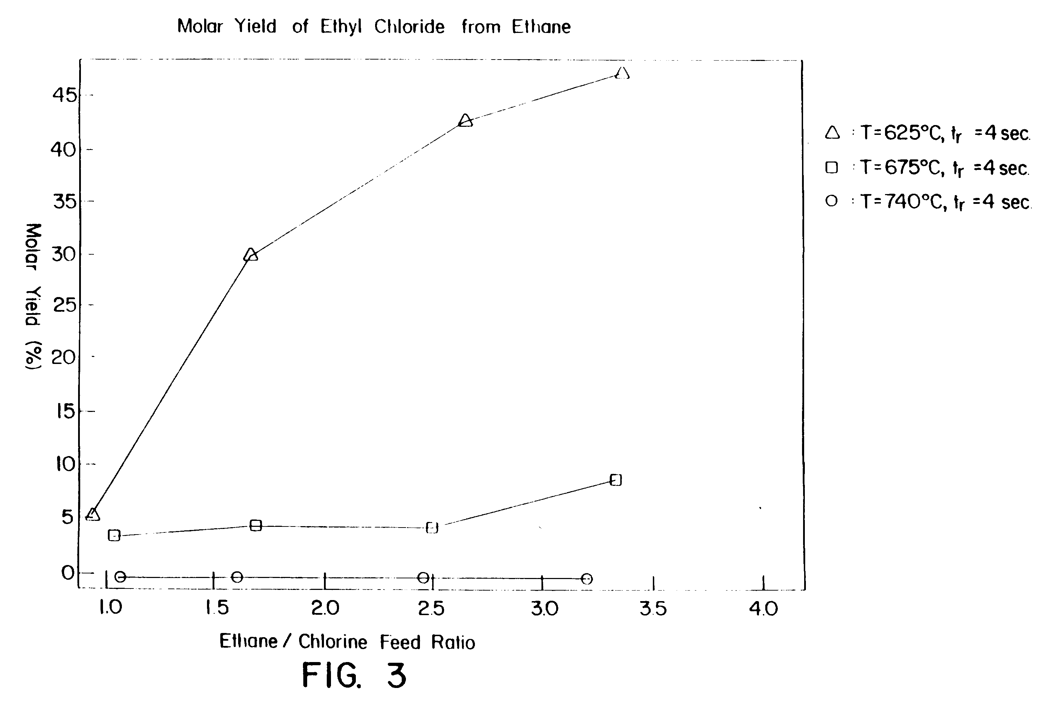

The yields of ethylene, vinyl chloride and ethyl chloride are plotted as a function of mole ratio and temperature in Figures 1, 2, and 3, respectively.

As can be seen from Table I and Figures 1-3, when the reaction temperature was above 650°C, the yield of vinyl chloride decreased with increasing ratio of ethane to chlorine in the feed. In addition, the yield of ethyl chloride increased as reaction temperature decreased and, at lower temperatures, also increased with increased ratio of ethane to chlorine in the feed. Consequently, at a temperature of 625°C good yields of ethylene and vinyl chloride were obtained only at an ethane to chlorine ratio of about 1:1.

Example 2

Employing the apparatus described in Example 1, a series of reactions was run at an ethane/chlorine feed ratio of 1.39:1 and a reactor temperature of 675°C, but retention time was varied from 3 to 11 seconds. Ethane consumption and yields of ethylene, vinyl chloride, and ethyl chloride were found to be as follows.

| | | Yields/% | | | |
|---|---|---|---|---|---|
| Reaction Time/Sec | Ethane Conversion/% | Ethylene | Vinyl Chloride | Ethylene +VCl | Ethyl Chloride |
| 2.9 | 69.8 | 71.7 | 17.3 | 89 | 6.6 |
| 3.7 | 69.8 | 72.3 | 17.9 | 90.2 | 5.9 |
| 7.3 | 70.5 | 79.5 | 16.2 | 95.7 | 0.2 |
| 11.0 | 68.8 | 79.5 | 15.7 | 95.2 | 0 |

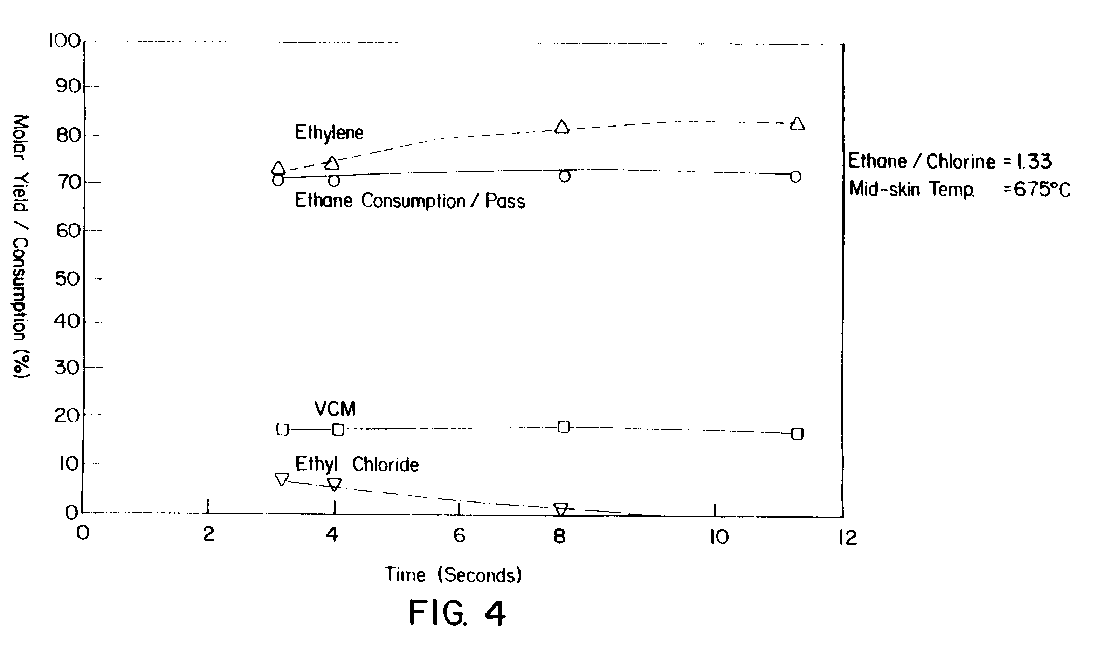

The dependence of ethane consumption and product yields is plotted as a function of reaction time in Figure 4.

As is evident, increased reaction time increased the yield of ethylene and reduced the yield of ethyl chloride, but had no significant effect on ethane conversion or vinyl chloride yield.

Comparative Example A

This example was intended to duplicate the experimental conditions given in Example 1 of U.S. Patent No. 2,259,195, with the exception that ethane was substituted for the butane used in the patent example. Sixty 1/hour of ethane and 120 1/hour of chlorine were premixed in a 2.2mm ID Teflon tube which was 50cm long. The mixture was passed through a 2mm quartz capillary tube against a frontal perforated plate heated externally by an electric furnace. The resulting reaction mixture passed through an air cooled section and a water cooled exchanger. The frontal plate was arranged in a 15mm inside diameter quartz tube. The skin temperature of the tube was measured. Reactor skin temperatures of 300, 600, and 800°C were tested. At all temperatures tested, large amounts of carbon were formed. In fact, carbon formation was so severe that in every case the reaction tubs was plugged with carbon less than a minute after gas flows were stabilized. Because of the short operation time, gas chromatographic analysis could not be performed to determine what other products, besides carbon, were formed in the reaction.

## Claims

1.  A process for preparing ethylene or a mixture of ethylene and vinyl chloride by reaction of ethane and chlorine which comprises:

    (a) providing a stream of ethane feed gas and a stream of chlorine feed gas;
    (b) thoroughly mixing said ethane and chlorine feed gases in a mixing zone at a molar ratio of ethane to chlorine of at least 0.9;
    (c) controlling the temperatures of said ethane feed gas and said chlorine feed gas so that substantially no chemical reaction takes place during and immediately after said mixing and such that the temperature of such mixture of gases immediately after mixing is less than 215°C;
    (d) passing said mixture into an inert reaction zone;
    (e) heating said mixture in said inert reaction zone to initiate chemical reaction between said ethane and said chlorine, this reaction being uncatalysed;
    (f) continuing the providing of heat to said reacting mixture as required so that the reacted mixture will have a temperature between 600°C and 800°C;

    such that the combined molar yield of ethylene and vinyl chloride is at least 80% of the ethane consumed.

2.  A process according to claim 1 wherein the temperature of said mixture is about ambient temperature.

3.  A process according to claim 1 wherein said reacted mixture has a temperature between 650°C and 750°C.

4.  A process according to claim 1 wherein said molar ratio of ethane to chlorine is between 1:1 to 4:1.

5.  A process according to claim 1 wherein said molar ratio of ethane to chlorine is between 1:1 to 2:1.

6.  A process according to claim 1 wherein said molar ratio of ethane to chlorine is between 1.3:1 to 1.6:1

7.  A process according to claim 1 wherein said reaction between said ethane and said chlorine is substantially completed within 1 minute after the commencement of said mixing.

8.  A process according to claim 1 wherein said reaction between said ethane and said chlorine is substantially completed within 10 seconds after the commencement of said mixing.

9.  A process according to claim 1 wherein said ethane and said chlorine are reacted in an inert reaction zone made of a material selected from quartz, silicon carbide, alumina, and graphite.

10. A process according to claim 1 further comprising reacting the ethylene and hydrogen chloride produced from the reaction of ethane and chlorine with an oxygen source to produce dichloroethane.

11. A process according to claim 10 further comprising heating said dichloroethane to produce vinyl chloride.

12. A process according to claim 1 wherein the reacted mixture has a temperature between 600°C and 800°C, and further comprising cracking the ethyl chloride in said reacted mixture to ethylene and hydrogen chloride.

13. A process according to claim 12 wherein said cracking is carried out at about 500°C in the presence of a dehydrohalogenation catalyst.

14. A process according to claim 1 wherein the ethane converted per pass through said reactor is at least 50% of the ethane in the feed.

**Patentansprüche**

1.  Verfahren zur Herstellung von Ethylen oder einer Mischung von Ethylen und Vinylchlorid durch Umsetzung von Ethan und Chlor, bei dem:

    (a) ein Ethaneinsatzmaterialgasstrom und ein Chloreinsatzmaterialgasstrom bereitgestellt werden,
    (b) die Ethan- und Chloreinsatzmaterialgase in einem Molverhältnis von Ethan zu Chlor von mindestens 0,9 in einer Mischzone gründlich gemischt werden,
    (c) die Temperaturen des Ethaneinsatzmaterialgases und des Chloreinsatzmaterialgases kontrolliert werden, so daß während und unmittelbar nach dem Mischen im wesentlichen keine chemische Reaktion stattfindet und die Temperatur einer solchen Mischung von Gasen unmittelbar nach dem Mischen niedriger als 215 °C ist,
    (d) die Mischung in eine inerte Reaktionszone geführt wird,
    (e) die Mischung in der inerten Reaktionszone erhitzt wird, um eine chemische Reaktion zwischen dem Ethan und dem Chlor zu initiieren, wobei diese Reaktion unkatalysiert ist,
    (f) die Lieferung von Hitze zu der Reaktionsmischung je nach Notwendigkeit fortgesetzt wird, so daß die umgesetzte Mischung eine Temperatur zwischen 600 °C und 800 °C hat,

    so daß die Gesamtmolausbeute an Ethylen und Vinylchlorid mindestens 80% des verbrauchten Ethans beträgt.

2.  Mischung nach Anspruch 1, bei dem die Temperatur der Mischung etwa Umgebungstemperatur ist.

3.  Verfahren nach Anspruch 1, bei dem die umgesetzte Mischung eine Temperatur zwischen 650 °C und 750 °C aufweist.

4.  Verfahren nach Anspruch 1, bei dem das Molverhältnis von Ethan zu Chlor 1 : 1 bis 4 : 1 beträgt.

5.  Verfahren nach Anspruch 1, bei dem das Molverhältnis von Ethan zu Chlor 1 : 1 bis 2 : 1 beträgt.

6.  Verfahren nach Anspruch 1, bei dem das Molverhältnis von Ethan zu Chlor 1,3 : 1 bis 1,6 : 1 beträgt.

7.  Verfahren nach Anspruch 1, bei dem die Reaktion zwischen dem Ethan und dem Chlor im wesentlichen innerhalb 1 Minute nach dem Beginn des Mischens vollständig abgelaufen ist.

8.  Verfahren nach Anspruch 1, bei dem die Reaktion zwischen dem Ethan und dem Chlor im wesentlichen innerhalb von 10 Sekunden nach dem Beginn des Mischens vollständig abgelaufen ist.

9.  Verfahren nach Anspruch 1, bei dem das Ethan und das Chlor in einer inerten Reaktionszone umgesetzt werden, die aus einem Material ausgewählt aus Quarz, Siliciumcarbid, Aluminiumoxid und Graphit hergestellt ist.

10. Verfahren nach Anspruch 1, bei dem das durch die Umsetzung von Ethan und Chlor erzeugte Ethylen und der durch die Umsetzung von Ethan und Chlor erzeugte Chlorwasserstoff ferner mit einer Sauerstoffquelle umgesetzt werden, um Dichlorethan zu erzeugen.

11. Verfahren nach Anspruch 10, bei dem ferner das Dichlorethan erhitzt wird, um Vinylchlorid herzustellen.

12. Verfahren nach Anspruch 1, bei dem die Reaktionsmischung eine Temperatur zwischen 600 °C und 800 °C aufweist und ferner das Ethylchlorid in der umgesetzten Mischung zu Ethylen und Chlorwasserstoff gecrackt wird.

13. Verfahren nach Anspruch 12, bei dem das Cracken bei etwa 500 °C in Gegenwart eines Dehydrohalogenierungskatalysators durchgeführt wird.

14. Verfahren nach Anspruch 1, bei dem das pro Durchlauf durch den Reaktor umgewandelte Ethan mindestens 50 % des Ethans in dem Einsatzmaterial ist.

**Revendications**

1.  Procédé pour préparer de l'éthylène ou un mélange d'éthylène et de chlorure de vinyle par réaction d'éthane et de chlore qui consiste:

(a) à fournir un courant d'alimentation d'éthane gazeux et un courant d'alimentation de chlore gazeux;

(b) à mélanger minutieusement lesdites alimentation d'éthane gazeux et de chlore gazeux dans une zone de mélange à un rapport molaire d'éthane au chlore d'au moins 0,9; (c) a réguler les temperatures de ladite alimentation d'éthane gazeux et de ladite alimentation de chlore gazeux de sorte que pratiquement aucune réaction chimique n'ait lieu pendant et immédiatement après ledit mélange, et de sorte que la température d'un tel mélange de gaz immédiatement après le mélange soit inférieure à 215°C;

(d) à faire passer ledit mélange dans une zone réactionnelle inerte;

(e) à chauffer ledit mélange dans ladite zone réactionnelle inerte pour amorcer la réaction chimique entre ledit éthane et ledit chlore, cette réaction n'étant pas catalysée;

(f) à continuer de chauffer ledit mélange réactionnel, si nécessaire, de façon que la température du mélange ayant réagi soit comprise entre 600°C et 800°C;

de sorte que le rendement molaire combiné d'éthylène et de chlorure de vinyle soit au moins de 80% de l'éthane consommé.

2. Procédé selon la revendication 1, dans lequel la température dudit mélange est aux environs de la température ambiante.

3. Procédé selon la revendication 1, dans lequel ledit mélange ayant réagi possède une température comprise entre 650°C et 750°C.

4. Procédé selon, la revendication 1, dans lequel ledit rapport molaire d'éthane au chlore est compris entre 1:1 et 4:1.

5. Procédé selon la revendication 1, dans lequel ledit rapport molaire d'éthane au chlore est compris entre 1:1 et 2:1.

6. Procédé selon la revendication 1, dans lequel ledit rapport molaire d'éthane au chlore est compris entre 1,3:1 et 1,6:1.

7. Procédé selon la revendication 1, dans lequel ladite réaction entre ledit éthane et ledit chlore est sensiblement achevée en 1 minute après le commencement audit mélange.

8. Procédé selon la revendication 1, dans lequel ladite réaction entre ledit éthane et ledit chlore est sensiblement achevée en 10 secondes après le commencement dudit mélange.

9. Procédé selon la revendication 1, dans lequel ledit éthane et ledit chlore sont mis à réagir dans une zone réactionnelle inerte faite d'une matière choisie parmi le quartz, le carbure de silicium, l'alumine et le graphite.

10. Procédé selon la revendication 1, consistant de plus à faire réagir l'éthylène et le chlorure d'hydrogène produits à partir de la réaction d'éthane et de chlore avec une source d'oxygène pour produire du dichloroéthane.

11. Procédé selon la revendication 10, consistant de plus à chauffer ledit dichloroéthane pour produire du chlorure de vinyle.

12. Procédé selon la revendication 1, dans lequel le mélange ayant réagi possède une température comprise entre 600°C et 800°C, et consistant de plus à craquer le chlorure d'éthyle dans ledit mélange ayant réagi en éthylène et chlorure d'hydrogène.

13. Procédé selon la revendication 12, dans lequel ledit craquage est effectué à environ 500°C en présence d'un catalyseur de déhydrohalogénation.

14. Procédé selon la revendication 1, dans lequel l'éthane converti par passage à travers ledit réacteur constitue au moins 50% de l'éthane dans l'alimentation.

Molar Yield of Ethylene from Ethane

□ : T=625°C, $t_r$ = 4 sec.
△ : T=675°C, $t_r$ = 4 sec.
○ : T=740°C, $t_r$ = 4 sec.

FIG.1

Molar Yield of VCM from Ethane

△ : T=625°C, $t_r$ = 4 sec.
□ : T=675°C, $t_r$ = 4 sec.
○ : T=740°C, $t_r$ = 4 sec.

Ethane/Chlorine Feed Ratio

FIG.2

EP 0 562 034 B1

Molar Yield of Ethyl Chloride from Ethane

△ : T=625°C, $t_r$ =4 sec.

□ : T=675°C, $t_r$ =4 sec.

○ : T=740°C, $t_r$ =4 sec.

FIG. 3

EP 0 562 034 B1

FIG. 4

FIG. 5

$Cl_2$ →[11]

$C_2H_6$ →[12]

MIXING DEVICE [10] →[13] REACTOR [14] →[15] HEAT EXCHANGER [16] →[17]

EP 0 562 034 B1